# EUROPÄISCHE PATENTSCHRIFT

(11) **EP 2 344 052 B1**
(45) Veröffentlichungstag und Bekanntmachung des Hinweises auf die Patenterteilung: **25.12.2013**
(21) Anmeldenummer: 09777982.1
(22) Anmeldetag: 19.08.2009
(51) Int. Cl.: A61B 17/221, A61B 17/22

(54) **VORRICHTUNG ZUR ERÖFFNUNG OKKLUDIERTER BLUTGEFÄSSE**
DEVICE FOR REOPENING OCCLUDED BLOOD VESSELS
DISPOSITIF POUR LA REOUVERTURE DES VESSEAUX BLOQUÉES

(30) Priorität: 19.08.2008 DE 102008038195
(43) Veröffentlichungstag der Anmeldung: 20.07.2011
(73) Patentinhaber: Phenox GmbH, 44801 Bochum (DE)
(72) Erfinder: MILOSLAVSKI, Elina, 70192 Stuttgart (DE); MONSTADT, Hermann, 44797 Bochum (DE); HANNES, Ralf, 44137 Dortmund (DE)
(74) Vertreter: Thiel, Christian
(86) Internationale Anmeldenummer: PCT/EP2009/006018
(87) Internationale Veröffentlichungsnummer: WO 2010/020408

(56) Entgegenhaltungen:
- EP-A1- 1 629 784
- EP-A2- 0 472 368
- WO-A2-2007/054307
- US-A1- 2008 109 032

## Beschreibung

Die Erfindung betrifft eine Vorrichtung zur Eröffnung okkludierter Blutgefäße, welche an einem Führungsdraht eine Geflechtstruktur aus einer Mehrzahl von Filamenten aufweist. Die Geflechtstruktur ist dabei fest und dauerhaft mit dem Führungsdraht verbunden.

Thromboembolische Erkrankungen wie Herzinfarkt, Lungenembolie, periphere Thrombose, Organembolien, etc. werden typischerweise durch einen Thromboembolus (im folgenden kurz Thrombus), also einen viskoelastischen Blutklumpen aus Blutplättchen, Fibrinogen, Gerinnungsfaktoren etc., ausgelöst, der sich in einem Blutgefäß festgesetzt hat und dieses ganz oder teilweise verschließt. Der Verschluß von Organarterien führt dabei zu einer Unterbrechung der Versorgung des abhängigen Gewebes mit Sauerstoff und Nährstoffen. Der Störung des Funktionstoffwechsels mit Funktionsverlust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thrombembolische Verschlüsse kommen gehäuft in den Bein- und Beckenvenen vor. Das Krankheitsbild des thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

So ist es bekannt, solche Patienten mit thrombolytischen Mitteln wie Streptokinase oder Urokinase oder mit Antikoagulantien zu behandeln, was zur Thrombolyse oder zur Eindämmung des Thrombenwachstums führen soll. Da diese Behandlungsmethoden meist zeitintensiv sind, werden sie oftmals zusammen mit Eingriffen kombiniert, die der mechanischen Zerkleinerung oder Entfernung des Thrombus bzw. Embolus dienen.

Neben offenen chirurgischen Eingriffen kommen im Stand der Technik zunehmend transluminale bzw. endovaskuläre, Katheter geführte interventionelle Therapieformen zum Einsatz, da diese weniger invasiv sind. So ist es bekannt, den Thrombus mittels Unterdruck erzeugenden Saug-Kathetern oder mechanisch mit Kathetern, welche mit Fangkörben, Wendeln, Haken, Bürsten oder dergleichen versehen sind, aus dem Körper des Patienten zu entfernen, siehe US 6 245 089 B1, US 5 171 233 A1, WO 2006/021407 A2, Thomas E. Mayer et al., Stroke 2002 (9), 2232.

Der Nachteil der bekannten transluminalen Vorrichtungen besteht darin, daß auch diese den Thrombus nicht immer oder nicht vollständig entfernen können, und die Gefahr besteht, daß der Thrombus oder Fragmente davon sich freisetzen und im Blutstrom zu kleinlumigeren Gefäßen weiterziehen, welche schwerer zu erreichen und zu behandeln sind. Des Weiteren führt der unterbrochene oder unzureichende Blutfluss zu gravierenden Folgeschäden, die mit der Zeitdauer der Unterbrechung zunehmen.

Bei dieser Situation besteht ein Bedarf an einer Vorrichtung, die es erlaubt, bei Thromben, die nicht oder nicht vollständig oder nicht schnell genug entfernt werden können, den Blutfluss zumindest teilweise und vorübergehend wieder herzustellen, um Zeit für einen interventionellen Eingriff zu bekommen, ggf. auch den Thrombus mit einer solchen Vorrichtung so zu beeinflussen, dass er ganz oder partiell entfernt werden kann.

Diese Aufgabe wird erfindungsgemäß mit einer Vorrichtung zur Eröffnung okkludierter Blutgefäße gelöst, welche an einem Führungsdraht eine damit fest und dauerhaft verbundene Geflechtstruktur aus einer Mehrzahl von Filamenten mit helixartigem Verlauf aufweist, die wenigstens am proximalen Ende zu einem Bündel zusammengeführt sind, wobei die Geflechtstruktur unter äußerem Zwang eine gestreckte Form kleineren Durchmessers annimmt und in entspanntem Zustand einen rohrförmigen Körper größeren Durchmesser definiert, der wenigstens an seinem proximalen Ende geschlossen ist, wobei die Vorrichtung eine Faserspannung aufweist.

Die erfindungsgemäße Vorrichtung wird in der Praxis in einem dafür geeigneten herkömmlichen Mikrokatheter an den Thrombus herangeführt. Der Katheter durchstößt dann den Thrombus und wird anschließend zurückgezogen, wobei die erfindungsgemäße Vorrichtung freigesetzt wird und sich aus dem unter den äußeren Zwang der Katheterwandung gespannten Zustand in einen entspannten Zustand größeren Durchmessers ausdehnt. In diesem Zustand definiert die Geflechtstruktur einen rohrförmigen Körper der sich soweit ausdehnt, wie es Gefäßwandung und/oder Thrombus zulassen. Die Vorspannung der Geflechtstruktur führt zu einer Kompression des Thrombus, ein zumindest partieller Blutfluss wird durch die Geflechtstruktur sichergestellt.

Gleichzeitig wirkt die Geflechtstruktur mit der Faserverspannung als Filterelement. Teile des Thrombus, die beim Aufspannen der Geflechtstruktur abgelöst werden, sammeln sich im Inneren der Geflechtstruktur am distalen Ende. Der Ablöseeffekt kann durch geeignete Bewegung der Geflechtstruktur (axiale oder Drehbewegung), die zu einem Schäleffekt führt, gefördert werden, so dass am Ende der Behandlung die Vorrichtung mit darin angesammelten Thrombusfragmenten in den Katheter zurückgezogen und aus dem Körper entfernt werden kann. Soweit der Thrombus nicht vollständig entfernt wird, bleibt zumindest ein Kanal offen, der die Durchblutung dahinterliegender Gefäßabschnitte erlaubt.

Erste Ergebnisse zeigen, dass die erfindungsgemäße Vorrichtung geeignet ist, Thromben in insbesondere auch kleinlumigen Gefäßen zu erreichen und zu durchstoßen. Der Führungsdraht ermöglicht eine gute Manövrierbarkeit auch in gewundenen Gefäßabschnitten und hält den Gesamtdurchmesser der Vorrichtung, insbesondere aber den nicht-variablen Durchmesser der Vorrichtung klein. Die Geflechtstruktur und die Fasern sind geeignet, Thrombusfragmente festzuhalten.

Die Fasern der Faserverspannung bestehen aus medizinisch geeigneten Fasermaterialien, beispielsweise Polyamid, Polyester oder Polypropylen. Polyamidfasern sind bevorzugt.

Als Faserverspannung wird eine räumliche Faserstruktur verstanden, die sich zumindest durch ein Teil des Innenraums der Geflechtstruktur erstreckt. Die Faserverspannung, die im Innern der Geflechtstruktur eine zusätzliche räumliche Netzstruktur ausbildet, dient dazu, bei der Expansion der Vorrichtung gelöste Teile des Thrombus zuverlässig zu sammeln und festzuhalten. Andererseits soll diese räumliche Netzstruktur offen genug sein, um einen Blutfluss von proximal nach distal durch die Geflechtstruktur hindurch zu ermöglichen.

Der Begriff "Faserverspannung" ist dahingehend zu verstehen, dass die Fasern von Filament zu Filament durch den Innenraum der Geflechtstruktur verlaufen. Eine gewisse Spannung der Fasern in vollexpandiertem Zustand der Geflechtstruktur ist möglich und kann sinnvoll sein, ist jedoch nicht Voraussetzung. Die Fasern können auch durchaus spannungslos im Innenraum verlaufen.

Der Vorteil der Faserverspannung gegenüber bekannten Bürstenstrukturen liegt darin, dass auf einem zentralen Draht, an dem die Borsten befestigt sind, verzichtet werden kann. Dies führt zu einer Volumenverminderung und damit zu einer räumlichen Verkleinerung insbesondere hinsichtlich des Durchmessers der Vorrichtung, die damit auch in feinere Gefäße eingeführt werden kann.

Da die erfindungsgemäße Vorrichtung nicht als Implantat konzipiert ist, versteht es sich, dass Führungsdraht und distales Element unlösbar miteinander verbunden sind. Die Vorrichtung wird zu gegebener Zeit, ggf. mit darin enthaltenen Fragmenten, in den Mikrokatheter oder einen weiteren Führungskatheter zurückgezogen und mit diesen aus dem Körper des Patienten entfernt.

Die Geflechtstruktur besteht aus einer Mehrzahl von helixartig verlaufenden Filamenten. Die Anzahl der verflochtenen Drähte kann sich in einem weiten Bereich bewegen, beispielsweise von 4 bis 128, jedoch sind im allgemeinen 6 bis 16 Filamente ausreichend. Die verflochtenen Drähte definieren im entspannten Zustand ein Rohr, dessen Wandung von den Filamenten gebildet wird, wobei in jedem Fall das proximale Ende des Rohres durch die zusammenlaufenden Filamente geschlossen ist, ggf. auch das distale Ende. Proximal bedeutet in diesem Zusammenhang, das zum behandelnden Arzt, zum Mikrokatheter bzw. zum Führungsdraht weisende Ende, distal das davon wegweisende Ende der Geflechtstruktur.

Die Geflechtstruktur besteht vorzugsweise aus Metallfilamenten, die Rückstell- bzw. Formgedächtniseigenschaften aufweisen. Es kann sich dabei um medizinischen Stahl mit guten Federeigenschaften handeln, insbesondere aber um Filamente aus Formgedächtnislegierungen wie beispielsweise Nitinol. Die Filamente haben insbesondere einen Durchmesser von 0,01 bis 0,2 mm.

Proximal wie distal sind die einzelnen Filamente zu einem Bündel zusammengefasst, das mit dem Führungsdraht fest, beispielsweise durch Verschweißen, Verkleben oder Verkrimpen, verbunden ist. Vorzugsweise wird das Bündel von einer Manschette zusammengehalten, die gleichzeitig als röntgendichter Marker dient und beispielsweise aus einem Platinmetall oder einer Platinmetalllegierung (Pt/Ir) besteht.

In der Regel wird das Filamentbündel zentral angeordnet sein, d. h. auf der Rotationsachse der Geflechtstruktur. In besonderen Fällen, beispielsweise aus Gründen der Manövrierbarkeit kann aber auch eine dezentrale Anordnung, etwa in Höhe der Wandung der Geflechtstruktur, sinnvoll sein.

Die Geflechtstruktur nimmt im Katheter eine durch den Katheterdurchmesser bestimmte gespannte und gestreckte Form kleineren Durchmessers an, in dem sie an den Einsatzort transportiert werden kann. Sobald die Geflechtstruktur aus dem Katheter freigesetzt wird, entspannt sie sich, soweit es die Umgebung zulässt, bis zu einem maximal möglichen größeren Durchmesser, der beispielsweise bis zu 10 mm betragen kann. In der Regel wird sich die Geflechtstruktur nicht vollständig entspannen und entfalten können, da sie zuvor an die Gefäßwandung und/oder an den Thrombus stößt. Dies ist auch sinnvoll, um die interne Spannung der Geflechtstruktur zur Kompression oder Teilung des Thrombus voll nutzen zu können. Die maximale Kraft, mit der sich die Geflechtstruktur gegen die Gefäßwand aufspannt, sollte in einer Richtung 0,3 N nicht übersteigen, um Verletzungen der Gefäßwand zu verhindern. Zweckmäßig sind 0,1 bis 0,3 N über eine Wirklänge von 20 mm.

Stent-ähnliche, geflochtene Strukturen - insbesondere mit distal und proximal verbundenen Geflechtdrahtenden - weisen bei regelmäßigen Flechtmustern unterschiedliche Radialkraftbereiche auf. Im statischen Ruhezustand ist in der Regel die Radialkraft in der Mitte schwächer ausgeprägt als in den Endbereichen. Dies liegt u. a. daran, dass sich die einzelnen Drähte im Flechtmuster über- und unterlaufen, ohne dass eine Verbindung zwischen ihnen besteht. Zudem werden die durch das Geflecht erzeugten Radialkräfte auf proximaler Seite durch Zugbewegung in proximaler Richtung reduziert, da die zentrale Verbindung die einzelnen Drähte in die Geflechtmitte zieht (Anwendung, dynamischer Zustand).

Werden einzelne, sich kreuzende Geflechtdrähte - insbesondere solche, die auf dem Umfang nebeneinander liegen - miteinander verbunden, so erhöht dies die Radialkraft des Gesamtgebildes. Es ist durchaus nicht erforderlich, dass dazu Verbindungen an allen Kreuzungspunkten erstellt werden. Dies würde auch die Flexibilität der Vorrichtung einschränken.

Durch die Zahl und Position der Verbindungen an den Kreuzungspunkten, bezogen auf Länge und Umfang, kann aber der Radialkraftverlauf des Geflechts gesteuert werden. Insbesondere im proximalen und mittleren Bereich ist eine Erhöhung der Radialkraft häufig wünschenswert. Zu diesem Zweck werden vorzugsweise im proximalen und mittleren Bereich des Geflechts, nicht jedoch am distalen Ende, an einzelnen Kreuzungspunkten von Filamenten Verbindungen hergestellt. Vorzugsweise befinden sich die miteinander verbundenen Kreuzungspunkte an Ringsegmenten am proximalen und/oder im bzw. bis zum mittleren Teil der Geflechtstruktur.

An den so zu verbindenden Kreuzungspunkten der Filamente können die Verbindungen über Schweißpunkte oder Klebpunkte hergestellt werden. Schweißpunkte können mittels Laserschweißen oder Widerstandspressschweißen erzeugt werden. Verlöten ist ebenfalls möglich.

Ferner können Verbindungen durch Umschlingen und/oder Verknoten der Kreuzungspunkte mit Draht oder Fadenmaterial hergestellt werden. Als Drahtmaterial kommt insbesondere Nitinol in Frage, aber auch Platin, Platinlegierungen und Edelstahl. Als Fadenmaterial können beispielsweise Polyamide verwandt werden. Vorteilhaft können die Fasern der Verspannung zu diesem Zwecke herangezogen werden.

Im allgemeinen sind die erfindungsgemäßen Geflechtstrukturen 10 bis 70 mm lang (Wirklänge) bei einem entspannten Außendurchmesser von 1 bis 10 mm, vorzugsweise 15 bis 30 mm lang bei einem Durchmesser von 3 bis 6 mm. Es ist sinnvoll, für verschiedene Gefäßdurchmesser entsprechende Durchmesser bei den Geflechtstrukturen vorzusehen.

Als Führungsdraht kann ein herkömmlicher Führungsdraht aus einem elastischen Material verwandt werden, beispielsweise einem medizinischen Stahl, der insbesondere am distalen Ende konisch zuläuft, etwa dünn geschliffen ist, um die Verbindung mit dem Filamentbündel und der Manschette zu erleichtern und um distal einen weicheren und flexibleren Draht bereitzustellen. Eine Abdeckung oder Beschichtung des Führungsdrahts, insbesondere im distalen Bereich, kann sinnvoll sein, beispielsweise ein aufgezogener PTFE-Schlauch.

Die erfindungsgemäße Vorrichtung weist radial durch den Innenraum laufende Fasern auf, die von Filament zur Filament verlaufen. Insbesondere verlaufen solche Fasern von Kreuzungspunkt zu Kreuzungspunkt der Filamente über die gesamte oder einen Teil der Wirklänge, insbesondere im distalen Teil.

Die Fasern können im Prinzip beliebig durch den Innenraum gespannt sein bzw. verlaufen. Bevorzugt ist aber die Anordnung in regelmäßigen Mustern, die, gesehen in Längsrichtung der Geflechtstruktur, strahlen- oder sternförmig sind. Damit lassen sich hinreichend dichte Netzwerke aus Fasern erzeugen, um Thromben zurückzuhalten, ohne dass der Blutfluss übermäßig behindert wird.

Bevorzugt sind Faserverspannungen, die im Zickzack längs durch die Geflechtstruktur verlaufen, wobei sie an gegenüberliegenden Kreuzungspunkten von Filamenten festgelegt sind und im Wesentlichen radial durch die Längsachse der Geflechtstruktur verlaufen. Weiterhin bevorzugt sind Anordnungen, wo die Fasern an nicht einander gegenüberliegenden Kreuzungspunkten von Filamenten festgelegt sind und senkrecht zur Längsachse der Geflechtstruktur, sternförmige regelmäßige Muster mit einer inneren Kanalstruktur ausbilden. In diesem Fall sind die Fasern beispielsweise, in einer Ebene gesehen, von einem Kreuzungspunkt jeweils zum übernächsten oder vierten geführt, usw., wobei jeweils die in einer Ebene liegenden Knotenpunkte alle einbezogen sind. Im ersten Fall ergibt sich durch die radial verlaufenden Fasern in Längsrichtung eine strahlenförmige Struktur mehrerer in Längsrichtung verlaufender Fasern, im zweiten Fall eine Anzahl von sternförmigen Strukturen in einer Mehrzahl von Ebenen der Geflechtstruktur.

Sowohl die Geflechtstruktur der Filamente als auch die darin verlaufenden/gespannten Fasern sind geeignet, als Filter zu wirken und vom Thrombus abgelöste oder abgeschälte Fragmente festzuhalten. Gleichzeitig ist die Vorrichtung durchlässig genug, um einen zumindest teilweisen Blutfluss durch den Thrombus hindurch über die gesamte Wirkzeit zu gewährleisten und damit die Durchblutung dahinterliegenden Gewebes sicherzustellen.

Zur Verbesserung der Filterwirkung kann es zweckmäßig sein, im distalen Bereich der Geflechtstruktur eine größere Dichte der Filamente vorzusehen, beispielsweise durch eine engere Führung und einen geringeren Abstand der in helixartigen Windungen geführten und ggf. verflochtenen Elemente oder aber durch die Anordnung zusätzlicher Filamente oder die Einarbeitung von Fasern in die Wandung der Geflechtstruktur. Eine solche Verdichtung der Geflechtstruktur kann beispielsweise nur in einem Teilbereich des geschlossenen Endes der Geflechtstruktur vorliegen, sich aber auch auf einen distalen Bereich der Geflechtstrukturwandung erstrecken.

Es versteht sich, dass die Geflechtstruktur mit der Faserverspannung, wie sie erfindungsgemäß mit einem Führungsdraht vorgesehen ist, ohne diesen Führungsdraht als temporäres oder längerfristiges Implantat eingesetzt werden kann. Der Führungsdraht kann hierzu ablösbar ausgelegt sein.

Die Erfindung wird durch die beiliegenden Abbildungen näher erläutert. Es zeigen:
- Figur 1: eine erfindungsgemäße Geflechtstruktur;
- Figur 2: eine Geflechtstruktur gemäß Fig. 1 mit eingespannter Faser;
- Figur 3: weitere Varianten einer erfindungsgemäßen Vorrichtung mit unterschiedlichem Faserverlauf;
- Figur 4: drei Varianten der Festlegung einer eingezogenen Faser an Kreuzungspunkten;
- Figur 5: Varianten einer distalverdichteten Geflechtstruktur;
- Figur 6: die Wirkweise der erfindungsgemäßen Vorrichtung;
- Figur 7: den Einfluss von Verbindungspunkten im Geflecht einer erfindungsgemäßen Vorrichtung auf die Radialkraft der Vorrichtung; und
- Figur 8: zwei Ausführungsformen einer erfindungsgemäßen Vorrichtung mit proximaler (a) und proximaler sowie zentraler (b) Anordnung der Verbindungspunkte.

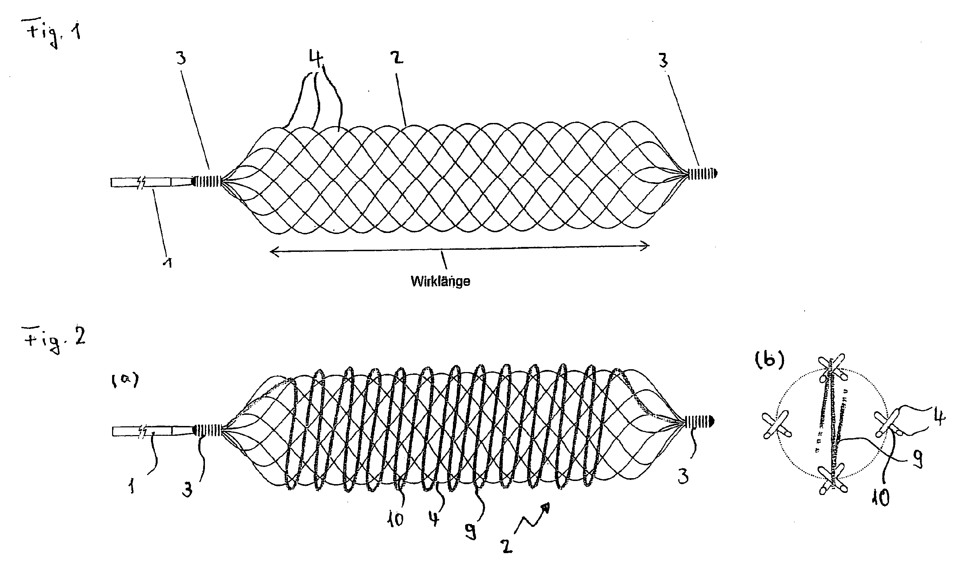

Figur 1 zeigt eine bevorzugte Ausführungsform der erfindungsgemäßen Vorrichtung mit einem Führungsdraht 1, einer Geflechtstruktur 2 sowie zwei röntgendichten Markern 3. Die Geflechtstruktur besteht aus insgesamt acht Filamenten 4, die bei im Wesentlichen parallelem Verlauf in helikalen Windungen einen rohrförmigen Körper definieren. Distal und proximal laufen die Filamente 4 in einem Bündel in der Rotationsachse der Geflechtstruktur zusammen, wobei beide Bündel von den röntgendichten Markern 3 als Manschette zusammengefasst werden. In der proximalen Manschette endet gleichzeitig der Führungsdraht 1, der zur Erhöhung der Flexibilität distal konisch zugeschliffen ist.

Die einzelnen Filamente 4 sind in allen Ausführungsformen vorzugsweise nach dem Muster "über 1/unter 1" miteinander verflochten, d. h. ein Filament 4 verläuft an seinen Kreuzpunkten mit anderen Filamenten abwechselnd oberhalb und unterhalb. "über 2/unter 2" ist ebenfalls möglich.

Die Geflechtstruktur 2 bleibt aufgrund der Filamentführung distal und proximal offen, während sie über ihre Länge von der daran angrenzenden Gefäßwand begrenzt wird. Der Blutfluss ist folglich in Längsrichtung der Geflechtstruktur durch diese Struktur hindurch möglich.

Figur 2a zeigt eine erfindungsgemäße Vorrichtung mit der Geflechtstruktur 2 und einzelnen Filamenten 4, die helixförmig verlaufen und miteinander verflochten sind. Zwischen den Markermanschette 3 verläuft eine Faser 9 von Kreuzungspunkt zu Kreuzungspunkt, die sich im Wesentlichen in einer Ebene durch das gesamte Geflecht 2 erstreckt.

Figur 2b zeigt die Anordnung schematisch im Querschnitt mit den Kreuzungspunkten der Filamente 4 und dem zwischen zwei Kreuzungspunkten verlaufenden Faden. Bei dem Faden bzw. Fasermaterial kann es sich um ein medizinisch erprobtes Material handeln, beispielsweise um ein Polyamid-, Polypropylen- oder Polyesterfilament.

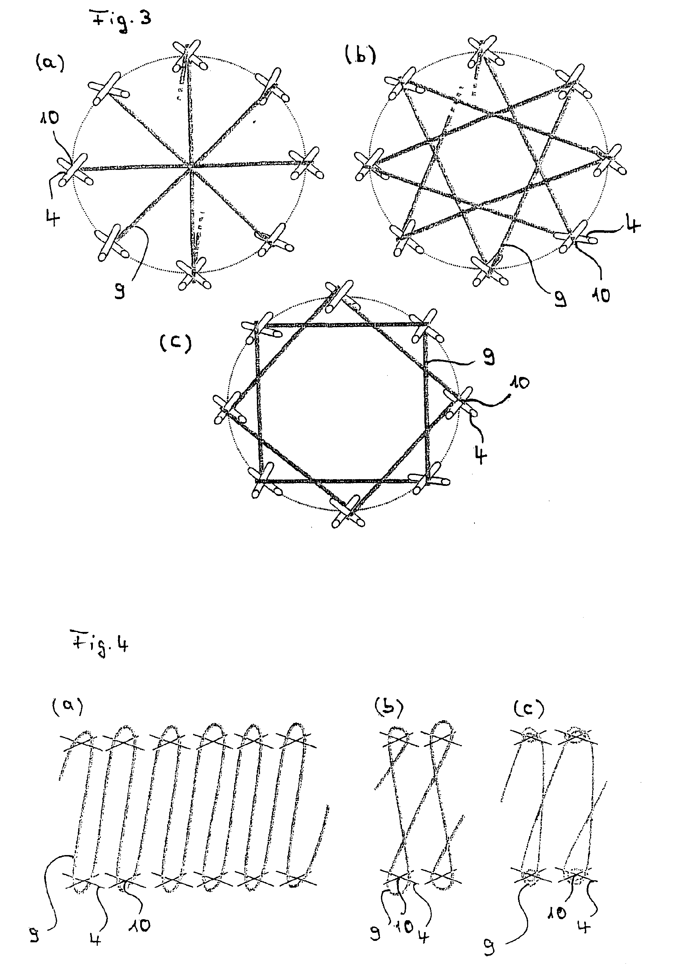

Figur 3 zeigt drei Varianten der Faserführung innerhalb einer Geflechtstruktur 2. Gemäß Fig. 3a ist der Faserverlauf längs, wobei die Fasern 9 von jeweils gegenüberliegenden Kreuzungspunkten 10 zweier Filamente 4 durch die Mitte der Geflechtstruktur von proximal nach distal verlaufen. Insgesamt vier des nach Art des in Fig. 2 gezeigten Faserverlaufs angeordnete Fasern ergeben insgesamt ein, in Längsrichtung der Vorrichtung gesehen, strahlenförmiges Muster.

Figur 3b zeigt einen Faserverlauf innerhalb einer Ebene der Geflechtstruktur 2, bei dem die Fasern von einem Kreuzungspunkt 10 zum jeweils vierten gespannt sind und innerhalb einer Ebene ein sternförmiges Muster mit einem zentralen Kanal ergeben. Die Fasern springen, nachdem die Kreuzungspunkte einer Ebene alle einbezogen sind, zu den Kreuzungspunkten der nächsten Ebenen, um dort das gleiche Muster zu erzeugen.

Figur 3c weist eine Struktur mit einem größeren inneren Kanal auf, bei dem die Fasern 9 von einem Kreuzungspunkt 10 zum übernächsten gespannt sind. Auch in diesem Fall ergibt sich ein sternförmiges Muster für jede Ebene. In diesem Fall können die Fasern nach Umlauf innerhalb einer Ebene wechseln; es ist aber auch möglich, jeweils nur die Kreuzungspunkte innerhalb einer Ebene mit einer oder mehreren Fasern zu bespannen und neue Fasern für die benachbarten Ebenen zu verwenden.

Es versteht sich auch, dass das dargestellte Verspannungsmuster sich über die gesamte Länge der Geflechtstruktur 2 erstrecken kann, andererseits aber auch nur eine Teillänge, vorzugsweise den distalen Bereich, erfüllen kann.

Figur 4 zeigt drei Varianten der Führung einer solchen Faser 9 zwischen Kreuzungspunkten von Filamenten 4 über einen Teil oder die gesamte Wirklänge einer Geflechtstruktur 2. Der Faden ist ½-mal um die Kreuzungspunkte herumgeführt (a). In Variante (b) ist der Faden einmal vollständig um den Kreuzungspunkt herumgeführt, was eine bessere Fixierung auch der Kreuzungspunkte der Filamente 4 bewirken. Gemäß Variante (C) umschlingt der Faden 9 einen jeden Kreuzungspunkt 10 der Filamente 4 1½-fach.

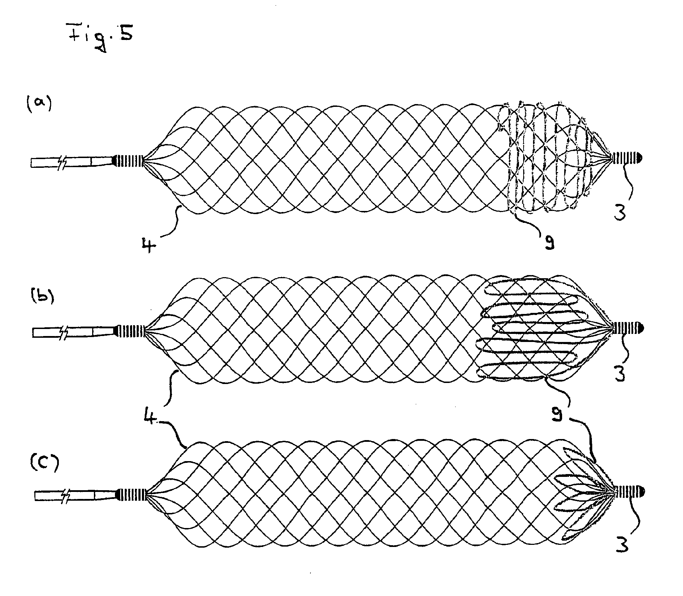

Figur 5 zeigt drei Varianten einer distalverdichteten Geflechtstruktur 2 mit in die Geflechtstruktur 2 distal eingearbeiteten Fasern 9 zur Verdichtung der "Hülle" und gemäß Figur 5a verläuft die Faser 9 abwechselnd über einem Filament 4, dann unter einem Filament 4. Gemäß Figur 5b verlaufen die Fasern 9 auf dem Umfang achsparallel hin und her und erzeugen so eine dichtere Hülle, wobei sie auch hier mit den Filamenten 4 verflochten sind.

Gemäß Figur 5c sind zusätzliche Drähte in die Markierungshülse 3 eingearbeitet, die schlaufenförmig verlaufen, wobei sich die Schlaufen blütenmäßig öffnen.

Es versteht sich, dass auch eine dichtere Verflechtung der Filamente 4 im distalen Bereich ohne Weiteres möglich ist.

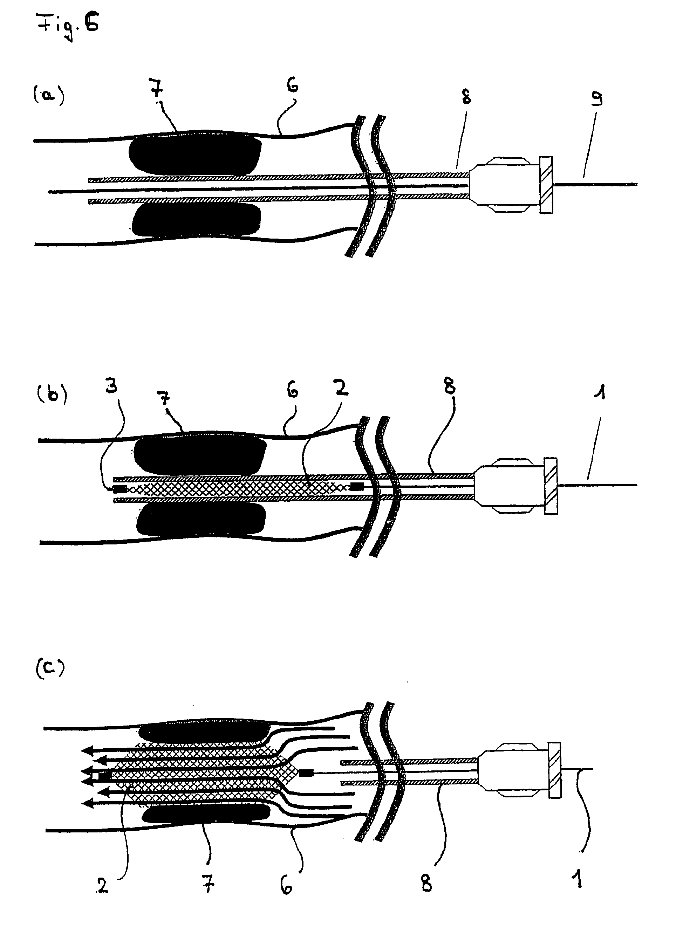

Figur 6 erläutert die Anwendungsweise der erfindungsgemäßen Vorrichtung. In einem Blutgefäß 6 befindet sich ein Thrombus 7, der bereits von einem Mikrokatheter 8 durchstoßen ist. Innerhalb des Mikrokatheters befindet sich ein Einführungsdraht 9, mit dessen Hilfe der Mikrokatheter zum Einsatzort manövriert wurde (Figur 6a).

Figur 6b zeigt dann innerhalb des Mikrokatheters 8 die erfindungsgemäße Geflechtstruktur 2 mit den distalen und proximalen Markern 3 am Führungsdraht 1. Die Geflechtstruktur befindet sich im gespannten und gestreckten Zustand, d. h. sie nimmt einen deutlich kleineren als den maximalen Durchmesser ein.

Figur 6c zeigt die erfindungsgemäße Vorrichtung nach Rückziehung des Mikrokatheters 8. Die Geflechtstruktur 2 hat sich innerhalb des Thrombus entspannt und aufgestellt und diesen an die Gefäßwand 6 gepresst. Durch die aufgespannte Geflechtstruktur 2 hindurch ist entlang der dargestellten Pfeile der Blutfluss wieder möglich.

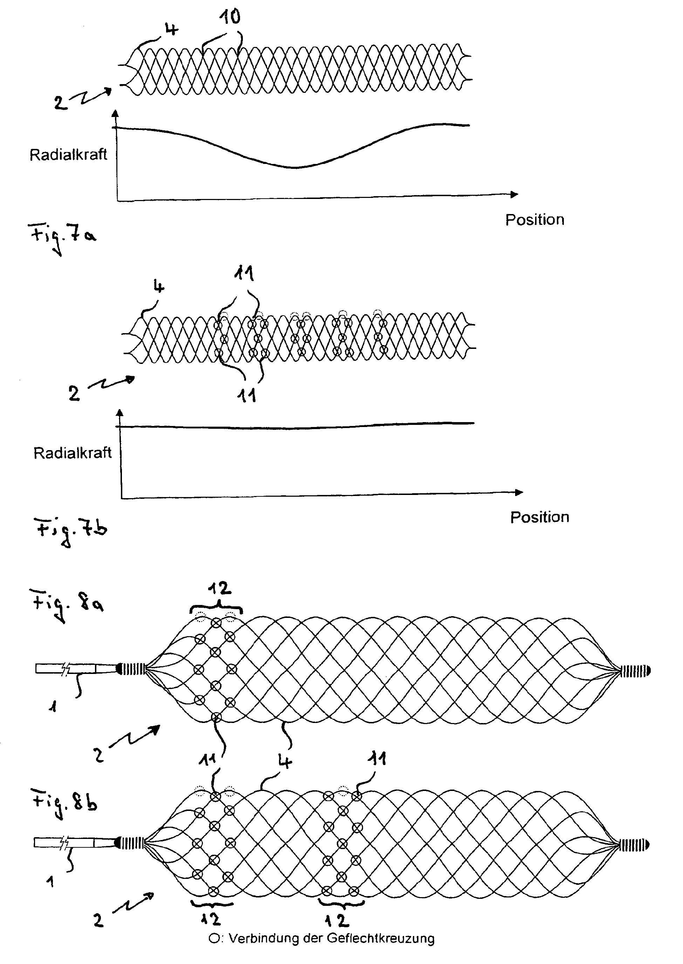

Figur 7 zeigt den Einfluss von an Kreuzungspunkten miteinander verbundenen Filamenten auf die Radialkraft eines Geflechts. Figur 7a zeigt das Geflecht 2 aus Filamenten 4 (nur die vordere Geflechtshälfte ist dargestellt) ohne miteinander verbundene Kreuzungspunkte 10. Bei maximaler Radialkraft an den verbundenen Enden des Geflechts nimmt die Radialkraft zur Mitte hin kontinuierlich ab, um dort einen Tiefpunkt zu bilden.

In Figur 7b ist die Radialkraft im mittleren Bereich durch die Einbringung von Verbindungen (z. B. Schweiß- oder Klebeverbindungen) 11 an Kreuzungspunkten 10 deutlich erhöht.

Figur 8a zeigt eine erfindungsgemäße Vorrichtung mit einem Führungsdraht 1, einer Geflechtstruktur 2 und Verbindungspunkten 11 an einigen Kreuzungspunkten des Geflechts. Die Verbindungen zwischen den Geflechtdrähten 4 sind nicht im einzelnen dargestellt, bestehen jedoch vorzugsweise aus Nylonfäden, die um die Drahtkreuzungen verknotet sind. Eine Befaserung der Vorrichtung, wie erfindungsgemäß vorgesehen ist nicht dargestellt.

Die Verwendung von Nylon, sowohl in den Kreuzungspunkten als auch bei der Befaserung, hat den Vorteil der Thrombogenizität, d. h., Thromben, die sich im Gefäß befinden, werden gebunden und können leicht entfernt werden.

In der Ausführungsform von Figur 8a befinden sich die Kreuzungspunkte 11 alle in einem ringförmigen Segment 12 am proximalen Ende der Vorrichtung. Figur 8b zeigt eine Variante, in der die Kreuzungspunkte in zwei ringförmigen Segmenten 12 konzentriert sind, nämlich am proximalen Ende der Vorrichtung und im mittleren Bereich. Die Verbindungspunkte, so wie sie auf dem Umfang verteilt sind, führen zu einem steiferen Zylinderabschnitt, was sowohl bei der Expansion der Vorrichtung wie auch bei der Extraktion eines Thrombus aus einem Gefäß durch Zurückziehen der Vorrichtung von Vorteil ist.

Es versteht sich, dass entsprechendes auch bei der Versteifung von geflochenen Stents mit Hilfe von Verbindungen an Kreuzungspunkten gilt. Bei Implantaten verbietet sich allerdings die Verwendung von thrombogenem Material.

Es versteht sich, dass das die Radialkraft erhöhende Design solcher Vorrichtungen nicht nur für erfindungsgemäße Vorrichtungen von Bedeutung ist, sondern ganz allgemein für Geflechte, die im Körper zum Einsatz kommen, beispielsweise geflochtene Stents, wie sie insbesondere im neuroradiologischen Bereich eingesetzt werden.

## Patentansprüche

1. Vorrichtung zur Eröffnung okkludierter Blutgefäße, welche an einem Führungsdraht (1) eine damit fest und dauerhaft verbundene Geflechtstruktur (2) aus einer Mehrzahl von Filamenten (4) mit helixartigem Verlauf aufweist, die wenigstens am proximalen Ende zu einem Bündel zusammengeführt sind, wobei die Geflechtstruktur (2) unter äußerem Zwang eine gestreckte Form kleineren Durchmessers annimmt und in entspanntem Zustand einen rohrförmigen Körper größeren Durchmesser definiert, der wenigstens an seinem proximalen Ende geschlossen ist, **dadurch gekennzeichnet, dass** die Vorrichtung eine Verspannung aus Fasern (9) aufweist, wobei die Fasern (9) innerhalb der Geflechtstruktur (2) zwischen Kreuzungspunkten (10) der Filamente (4) verlaufen.

2. Vorrichtung nach Anspruch 1 mit 6 bis 16 Filamenten (4).

3. Vorrichtung nach Anspruch 1 oder 2, **dadurch gekennzeichnet, dass** die Filamente (4) aus einer Formgedächtnislegierung bestehen, vorzugsweise aus Nitinol.

4. Vorrichtung nach einem der vorstehenden Ansprüche, **dadurch gekennzeichnet, dass** die Filamente (4) zumindest am proximalen Ende zentral oder exzentrisch zusammengeführt sind.

5. Vorrichtung nach Anspruch 4, **dadurch gekennzeichnet, dass** die Filamente in Manschetten (3) zusammengeführt sind, die zugleich als röntgendichte Marker ausgebildet sind.

6. Vorrichtung nach einem der vorstehenden Ansprüche, **dadurch gekennzeichnet, dass** die Geflechtstruktur (2) distal verdichtet ist, vorzugsweise durch eingearbeitete Fasern (2).

7. Vorrichtung nach einem der vorstehenden Ansprüche, **dadurch gekennzeichnet, dass** die Faserverspannung nur im distalen Bereich der Geflechtstruktur vorhanden ist.

8. Vorrichtung nach einem der vorstehenden Ansprüche, **dadurch gekennzeichnet, dass** die Fasern (9) innerhalb der Geflechtstruktur (2) eine räumliche Struktur ausbilden.

9. Vorrichtung nach einem der vorstehenden Ansprüche, **dadurch gekennzeichnet, dass** die Fasern (9) im Zickzack zwischen einander gegenüberliegenden Kreuzungspunkten (10) von Filamenten (4) längs durch die Geflechtstruktur (2) verlaufen.

10. Vorrichtung nach einem der Ansprüche 1 bis 9, **dadurch gekennzeichnet, dass** die Fasern (9) zwischen nichtbenachbarten Kreuzungspunkten (10) und Filamenten (4) in einer Ebene senkrecht zur Längsrichtung der Geflechtstruktur (2) verlaufen.

11. Vorrichtung nach einem der vorstehenden Ansprüche, **dadurch gekennzeichnet, dass** die Geflechtstruktur (2) auf eine Kraft, in Richtung auf eine Gefäßwand, von maximal 0,3 N über eine Wirklänge von 20 mm eingestellt ist.

12. Vorrichtung nach einem der vorstehenden Ansprüche, **dadurch gekennzeichnet, dass** die Geflechtstruktur (2) an Kreuzungspunkten (10) miteinander verbundene Filamente (4) aufweist.

13. Vorrichtung nach Anspruch 12, **dadurch gekennzeichnet, dass** sie die miteinander an Kreuzungspunkten (10) verbundenen Filamente (4) im proximalen und/oder mittleren Bereich aufweist.

14. Vorrichtung nach Anspruch 12 oder 13, **dadurch gekennzeichnet, dass** die Filamente (4) an Kreuzungspunkten (10) miteinander verschweißt oder verklebt oder mit zusätzlichem Faser- oder Drahtmaterial verknotet sind.

## Claims

1. Device for opening occluded blood vessels, comprising a braided structure (2) on a guide wire (1), said braided structure (2) being securely and permanently attached to the guide wire and consisting of a plurality of filaments (4) arranged in a helical manner and at least at the proximal end combined into a bundle, with the braided structure (2) assuming an elongated form of smaller diameter when subjected to external force while when in unstressed state defining a tubular element of larger diameter closed at least at its proximal end, **characterized in that** the device is provided with restraining/bracing fibers (9), the fibers (9) within the braided structure (2) extending between points of intersection (10) of filaments (4).

2. Device according to claim 1 comprising 6 to 16 filaments (4).

3. Device according to claim 1 or 2, **characterized in that** the filaments (4) consist of a shape-memory alloy, in particular of nitinol.

4. Device according to any of the above claims, **characterized in that** the filaments (4) at least at the proximal end converge centrically or eccentrically.

5. Device according to claim 4, **characterized in that** the filaments terminate in sleeves (3) which are also designed to serve as radiopaque markers.

6. Device according to any of the above claims, **characterized in that** the braiding structure (2) is of densified configuration distally, preferably through incorporated fibers (2).

7. Device according to any of the above claims, **characterized in that** the restraining fibers are arranged only in the distal area of the braided structure.

8. Device according to any of the above claims, **characterized in that** the fibers (9) within the braided structure (2) form a spatial structure.

9. Device according to any of the above claims, **characterized in that** the fibers (9) extend in zig-zag fashion between oppositely arranged points of intersection (10) of filaments (4) longitudinally through the braiding structure (2).

10. Device according to any of the claims 1 to 10, **characterized in that** the fibers (9) extend between non-neighboring points of intersection (10) and filaments (4) in a plane perpendicular to the longitudinal direction of the braided structure (2).

11. Device according to any of the above claims, **characterized in that** the braided structure (2) is adjusted to a force exerted towards a vessel wall of maximum 0.3 N over an effective length of 20 mm.

12. Device according to any of the above claims, **characterized in that** the braided structure (2) has filaments (4) connected with each other at points of intersection (10).

13. Device according to claim 12, **characterized in that** it has been provided in the proximal and/or central area with filaments (4) connected with each other at points of intersection (10).

14. Device according to claim 12 or 13, **characterized in that** the filaments (4) at points of intersection (10) are welded with or bonded to each other or knotted together by means of additional fiber or wire material.

## Revendications

1. Dispositif permettant d'ouvrir des vaisseaux occlus, lequel comporte, au niveau d'un fil de guidage (1), une structure tressée (2) reliée de manière fixe et durable à ce dernier et formée d'une pluralité de filaments (4) à tracé hélicoïdal, qui sont réunis au moins au niveau de l'extrémité proximale pour former un faisceau, ladite structure tressée (2) étant amenée, sous l'effet d'une force extérieure, dans une forme étirée avec un plus petit diamètre et définissant, à l'état détendu, un corps tubulaire avec un plus grand diamètre, qui est fermé au moins au niveau de son extrémité proximale, **caractérisé en ce que** ledit dispositif comporte un haubanage en fibres (9), lesdites fibres (9) s'étendant à l'intérieur de la structure tressée (2) entre les points d'intersection (10) des filaments (4).

2. Dispositif selon la revendication 1, comportant 6 à 16 filaments (4).

3. Dispositif selon la revendication 1 ou 2, **caractérisé en ce que** les filaments (4) sont réalisés dans un alliage à mémoire de forme, de préférence en nitinol.

4. Dispositif selon l'une quelconque des revendications précédentes, **caractérisé en ce que** les filaments (4) sont réunis de manière centrée ou excentrique, au moins au niveau de l'extrémité proximale.

5. Dispositif selon la revendication 4, **caractérisé en ce que** les filaments sont réunis en manchettes (3) qui sont réalisées en même temps sous forme de repères radio-opaques.

6. Dispositif selon l'une quelconque des revendications précédentes, **caractérisé en ce que** la structure tressée (2) est compactée en distal, de préférence par des fibres (2) incorporées.

7. Dispositif selon l'une quelconque des revendications précédentes, **caractérisé en ce que** le haubanage par fibres est présent uniquement dans la zone distale de la structure tressée.

8. Dispositif selon l'une quelconque des revendications précédentes, **caractérisé en ce que** les fibres (9) forment une structure tridimensionnelle à l'intérieur de la structure tressée (2).

9. Dispositif selon l'une quelconque des revendications précédentes, **caractérisé en ce que** les fibres (9) s'étendent en zigzag dans le sens longitudinal à travers la structure tressée (2), entre des points d'intersection (10), face à face, de filaments (4).

10. Dispositif selon l'une quelconque des revendications 1 à 9, **caractérisé en ce que** les fibres (9) s'étendent entre des points d'intersection (10) non adjacents et des filaments (4) dans un plan perpendiculaire à la direction longitudinale de la structure tressée (2).

11. Dispositif selon l'une quelconque des revendications précédentes, **caractérisé en ce que** la structure tressée (2) est réglée à une force de 0,3 N maximum sur une longueur d'action de 20 mm, appliquée dans la direction vers une paroi du vaisseau sanguin.

12. Dispositif selon l'une quelconque des revendications précédentes, **caractérisé en ce que** la structure tressée (2) comporte, au niveau des points d'intersection (10), des filaments (4) reliés les uns aux autres.

13. Dispositif selon la revendication 12, **caractérisé en ce que** ledit dispositif comporte, dans la zone proximale et/ou la zone centrale, les filaments (4) reliés les uns aux autres au niveau des points d'intersection (10).

14. Dispositif selon la revendication 12 ou 13, **caractérisé en ce que**, au niveau des points d'intersection (10), les filaments (4) sont soudés ou collés les uns aux autres ou sont noués à des fibres ou à un matériau en fil supplémentaires.
